# EUROPEAN PATENT SPECIFICATION

(11) **EP 1 150 661 B1**
(45) Date of publication and mention of the grant of the patent: **22.10.2003**
(21) Application number: 00902727.7
(22) Date of filing: 07.02.2000
(51) Int. Cl.: A61K 9/70, A61K 9/12

(54) **TOPICAL SPRAYS COMPRISING A FILM FORMING COMPOSITION**
TOPISCHE SPRAYS ENTHALTEND EINE FILMBILDENDE ZUSAMMENSETZUNG
SPRAYS TOPIQUES CONTENANT UNE COMPOSITION FILMOGENE

(30) Priority: 05.02.1999 IN BO009299; 05.02.1999 IN BO009399; 20.05.1999 IN BO038299; 17.08.1999 IN BO058299; 09.09.1999 WO PCT/GB99/02998; 13.01.2000 IN BO432000; 13.01.2000 IN BO442000
(43) Date of publication of application: 07.11.2001
(73) Proprietor: Cipla Ltd., Mumbai 400 008 (IN)
(72) Inventor: LULLA, Amar, Colaba Mumbai 400 005 (IN); MALHOTRA, Geena, Mazgaon Mumbai 400 010 (IN); RAUT, Preeti, Vile Parle (East) Mumbai 400 057 (IN)
(74) Representative: Wain, Christopher Paul
(86) International application number: GB0000366
(87) International publication number: WO00045795

(56) References cited:
- EP-A- 0 055 396
- EP-A- 0 055 397
- EP-A- 0 521 455
- EP-A- 0 617 972
- EP-A- 0 679 390
- EP-A- 0 756 870
- WO-A-88/09185
- WO-A-96/30000
- US-A- 4 826 677

## Description

This invention relates to topical medicinal spray compositions and to their preparation, which compositions can be used to treat a variety of disorders.

Many delivery systems for the topical application of pharmaceutical compounds are currently available and include lotions, creams, gels, ointments, transdermal patches and sprays. The choice of delivery system usually depends upon the desired pharmacokinetic profile of the drug, for example whether immediate - or sustained - release is required. Many of these systems suffer from occlusion problems and may cause skin irritation. For example, many compounds, including hormonal drugs, are conventionally delivered using a transdermal patch. These patches comprise an occlusive backing membrane which often results in local skin irritation. A further disadvantage of transdermal patches in that percutaneous penetration of the drug is often poor.

Topical spray formulations can help reduce the problem of skin irritation associated with transdermal patches. For example, British patent specification no. 1,372,721 discloses a container of antiseptic for the topical treatment of burns and scalds, containing a topically acceptable antiseptic active agent against *Pseudomonas aeruginosa,* a pressuring agent and at least one surfactant admixed with water. The container comprises an outlet, and valve means operable to allow discharge of the contents of the container through the outlet in the form of a foam which is effective in the control of *Pseudomonas aeruginosa* at the site of a burn or scald. US patent specification no. 4,534,958 describes and claims "a sprayable aerosol foam treatment composition which is a liquid in the aerosol container and forms a gel upon application to the skin", which composition comprises water, propellant, volatile solvent, and a polyoxyethylene-polyoxypropylene copolymer whose function is not described and optionally including a burn treatment agent and one or more adjuvants. The composition is used "for treating living skin".

WO 88/09185 discloses a pharmaceutical dressing comprising a film-forming polymer and an active: the polymer matrix forms a flexible film on hardening on the skin. EP-A-0055397 and EP-A-0055396 are further examples of this type of composition, and describe compositions comprising medicament in a volatile vehicle along with a film-forming polymer.

EP-A-756870 discloses a pharmaceutical spray composition based specifically on ketoprofen, and comprises specific quantities of active, alcohol, propylene glycol, poloxamer, polyvinylpyrrolidone, ethanolamine and water.

US 4826677 describes a composition for the local and nonirritating treatment of psoriasis. The compositions are primarily water-based and comprise film-forming polymers and antipsoriatic agents.

WO 96/30000 describes a transdermal delivery composition containing an optional polymeric release matrix made of cellulose polymers or copolymers and vinylpyrrolidone/vinyl acetate copolymers; active; an absorption promoter; and a non-aqueous solvent.

EP-A-521455 discloses an aerosol composition for locally treating damaged skin, and containing a biodegradeable hydroxycarboxylic acid polymer as a film-forming component. After spraying on the skin, the polymer is capable of being degraded or decomposed by natural effectors.

However, a problem with conventional topical spray formulations is that they tend to remain for only a short time at the application site - for example they are easily rubbed off. In consequence the medicament to be absorbed through the skin is only available transiently. By contrast, medicament in a transdermal patch is potentially available for as long as the patch remains in place.

We have now found a way of combining the advantages offered by transdermal patches and topical sprays, whilst reducing or minimising the disadvantages associated with each. We have devised a topical spray composition which can be sprayed on to the skin to form a breathable film or patch, which film remains stable and in place over a period of days. In this way medicament can, for example, be delivered transdermally over a period of time. Since the film is non-occlusive the problem of local skin irritation associated with transdermal patches is thereby substantially reduced.

According to the present invention, there is provided a topical, medicinal spray composition comprising one or more medicaments in a volatile vehicle, one or more film-forming polymers in said vehicle, and from 1 to 10% (w/w) plasticiser, said plasticiser comprising dimethyl isosorbide, wherein the composition can be sprayed on a topical site to form a stable, breathable film on said site, from which film the medicaments are transdermally available.

There is also provided a dispenser containing the composition of the invention, which dispenser dispenses the composition as a spray.

EP-A-0679390 describes sprayable compositions for treating the skin or a mucous membrane, the compositions having an active ingredient and a gel-forming cellulosic polymer. The active ingredients described are antiviral agents, antiseptics and local anaesthetics, and their use for treating cold sores is particularly exemplified. These actives are principally surface acting whereas, in the compositions of the present invention, the active is preferably for transdermal passage to act generally systemically.

EP-A-761095 describes antimicrobial film-forming compositions containing iodine. In general, the compositions of the present invention will be iodine-free, and will not usually contain any surface acting antimicrobial such as iodine. Furthermore, the compositions of EP-A-761095 contain at least 65% ethanol whereas, in general, the compositions of the present invention will contain at most about 60% ethanol and usually much less.

Preferably, the compositions of the invention also contain a permeation enhancer, and such a composition preferably contains up to 30% by weight of said medicament(s), up to 8% by weight of said permeation enhancer and up to 90% by weight of said vehicle. The amount of film-forming polymer is preferably up to about 15% by weight.

Preferred compositions of the invention comprise from 0.1% to 25% of one or more medicaments, from 0.1% to 10% film-forming polymer and said vehicle q.s. 100%, and further comprising from 0.1% to 10% solubilizer, from 0.1% to 8% permeation enhancer from about 1% to 10% plasticiser.

In the films provided by the present spray composition, the or each medicament is deposited in the matrix of the film-forming polymer(s) and may be in solution or in the form of a suspension. Depending upon the polymers chosen, the medicament may be released from the film over a period of time (i.e. sustained release) or immediately. The medicament so delivered by the film can be used to treat topical medical complaints and complaints which are local to the site of the film, but is preferably for systemic medical conditions which require transdermal delivery of the medicament into the bloodstream. The present topical spray composition can be used in the treatment of both humans and animals.

Preferably, the composition further comprises from 1% to 7% (w/w) of one or more water-soluble additives. The composition can be dispensed from any dispenser which provides the composition as a spray.

Accordingly, the present invention also provides a dispenser containing the spray composition of the invention, which dispenser dispenses the composition as a spray.

Preferably, the composition is dispensed from a pump dispenser or from an aerosol dispenser. In the latter case, the composition additionally comprises from 10% to 90% of propellant in order to provide a suitable pressure within the aerosol dispenser. Generally, propellant is not required for compositions dispensed from a pump dispenser. However, if desired, such compositions may also comprise from 10% to 90% of a propellant which is liquid at room temperature, for example trichloromonofluoromethane (P11).

In another aspect, the invention provides a method of preparing a pump dispenser containing the spray composition of the invention, which method comprises mixing the ingredients of the composition with or without liquid propellant, and then placing the mixed ingredients in a pump dispenser.

In a further aspect, the invention provides a method of preparing an aerosol dispenser containing the spray composition of the invention, which method comprises mixing the ingredients of the composition without propellant, and then charging the mixture together with propellant into an aerosol dispenser.

The composition is preferably dispensed from the chosen dispenser in a metered dose.

The medicament can be any medicinal compound, preferably in salt or base form, which is stable on mixing with the other ingredients of the composition and effective on topical administration; or a combination of any two or more such compounds. Preferably the medicament is a drug which is anti-emetic, anti-anginal, anti-inflammatory, a steroid, a steroid hormone, a bronchodilator or a drug used to treat osteoporosis. Additional preferred medicaments include drugs used to treat incontinence, antidepressants/anxiolytics, antimigraine agents, agents used in smoking cessation therapy, antidiarrhoeas, antiulcerants, anticholinergics, anticonvulsants, drugs for mood disorders/obsessive compulsive disorder, ACE inhibitors, calcium channel blockers, antihypertensives/diuretics, antiobesity drugs, hormonal peptides and analogues, drugs for benign prostatic hyperplasia/urinary retension and erectile dysfunctions, antiparkinson agents such as dopamine agonists and MAO inhibitors, drugs for sleep disorders and antidiabetic agents.

One preferred anti-emetic is scopolamine. Preferred anti-anginals include nitroglycerine, clonidine, isosorbide dinitrate, propranolol hydrochloride, timolol maleate, clonazepam or verapamil. Preferred anti-inflammatory drugs include diclofenac sodium, alendronate sodium, ibuprofen, ketoprofen, indomethacin piroxicam, ketorolac, tromethamine or nimesulide. Preferred steroids include hydrocortisone and esters thereof, dexamethasone, fluocinolone acetonide or betamethasone and salts thereof. Preferred hormonal steroids include estradiol or noethisterone or a combination thereof, testosterone or progesterone. Preferred bronchodilators include salbutamol base and its salts and bambuterol, salmeterol xinafoate, fluticasone propionate, mometasone furoate, budesonide, beclomethasone dipropionate, sodium cromoglycate, or isoprenaline sulphate. Preferred drugs for treating osteoporosis include alendronic acid, pamidromic acid, etidromic acid and their pharmaceutically acceptable salts. Preferred drugs used to treat incontinence include vasopressin and oxybutynin. Preferred antidepressants/anxiolytics include imipramine, Mirtazapine and desipramine. Preferred antimigraine agents include naratriptan, zolmitriptan and sumatriptan. One preferred antidiarrhoeal is loperamide. One preferred antiulcerant is misoprostol. Preferred anticholinergics include hyoscyamine, atropine and trihexyphenidyl. Preferred anticonvulsants include lorazepam, diazepam and tiagabine. Preferred drugs for antimood disorders/obsessive compulsive disorder include fluoxetine and paroxetine. Preferred ACE inhibitors include lisinopril, trandolapril and captopril. Preferred calcium channel blockers include amlodipine and felodipine. Preferred antihypertensives/diuretics include prazosin and amiloride. Preferred antiobesity drugs include methamphetamine and sibutramine hydrochloride. Preferred hormonal peptides and analogues include GnRH analogues such as nafarelin; leuprolide acetate, insulin and growth hormone and analogues thereof. Preferred drugs for benign prostatic hyperplasia/uninary retention include doxazosin, tamsulosin, terazosin and finasteride. Preferred drugs for erectile dysfunction include alprostadil and sildenafil citrate. Preferred antiparkinson agents include dopamine agonists such as bromocriptine and cabergoline and MAO inhibitors such as selegiline HCl. One preferred agent for sleep disorders is melatonin. Preferred antidiabetic agents include 1st and 2nd generation sulphonyl ureas such as glimepiride, rosiglitazone, glyburide and glipizide. Also, the chiral forms of all the drugs mentioned above can be used in the topical spray composition of the present invention.

The film-formers preferably include any acrylic polymers or copolymers. Preferred film-formers include a non-ionic copolymer of methyl methacrylate and butyl methacrylate (Plastoid B®), a copolymer of dimethylamine ethyl methacrylate and a neutral methacrylic acid ester (Eudragit E100®), ammonio methacrylate copolymer type B (Eudragit RS®, USP/NF), ammonio methacrylate copolymer type A (Eudragit RL®, USP/NF), methacrylic acid copolymer type A (Eudragit L100®, USP/NF), methacrylic acid copolymer type B (Eudragit S100®, USP/NF), polyvinyl acetate, cellulose acetate, polyvinyl alcohol, povidone, povidone vinyl acetate, hydroxypropyl methyl cellulose, hydroxy ethyl cellulose and methyl cellulose.

The breathability of the film is achieved by the absence of any occlusive backing membrane together with the generally hydrophilic properties of the film-forming polymer(s). These polymers can partially dissolve on exposure to moisture (from the skin or air), which dissolution results in the development of a porous film. This porosity can be enhanced by including further water-soluble additives, such as those detailed below.

Preferred solubilizers include a copolymer of dimethylamine ethyl methacrylate and a neutral methacrylic acid ester (Eudragit E100®, USP/NF); surfactants, for example, sodium lauryl sulphate; polyhydric alcohols, for example, propylene glycol or polyethylene glycols; vitamin E, vitamin E TPGS (tocopheryl polyethylene glycol 1000 succinate) and labrasol; or any two or more of the above in combination. Preferably, the solubilizer is a copolymer of dimethylamine ethyl methacrylate and a neutral methacrylic acid ester (Eudragit E100®) in combination with, a non-ionic copolymer of methyl methacrylate and butyl methacrylate (Plastoid B®). The solubilizers serve to dissolve or suspend the drug in the chosen vehicle. Thus, in a topical film formed by spraying the present composition, the medicament may be present in a partially or completely dissolved form or in the form of a suspension. Many of the solubilizers also enhance percutaneous penetration of drug and/or act as humectants.

Preferred plasticisers include triethyl citrate, dimethyl isosorbide, acetyltributyl citrate, castor oil, propylene glycol, and polyethylene glycol, or any two or more of the above in combination.

The permeation enhancer is preferably a lyophilic solvent, for example, dimethyl sulfoxide, dimethyl formamide or isopropyl myristate; a surfactant, for example, Tween 80, sodium lauryl sulfate or menthol; ; a two-component system, for example, oleic acid and octyl dimethyl paraamino benzoic acid (Padimate O); or a polyhydric alcohol, for example, propylene glycol or diethylene glycol monoethyl ether EP (transcutol); or any two or more of the above in combination.

The vehicle can be water or a non-aqueous solvent. Preferred non-aqueous vehicles include acetone, isopropyl alcohol, methylene chloride, methyl-ethyl-ketone, absolute alcohol, ethyl acetate and trichloromonofluoromethane (P11); or any two or more of the above in combination.

The aqueous or non-aqueous vehicle may additionally comprise (weight/weight of vehicle) from 1% to 20% of one or more humectants. Preferred humectants include polyhydric alcohols and polyvinylpyrrolidone. Preferred polyhydric alcohols are propylene glycol, butylene glycol, polyethylene glycols, glycerol and sorbitol.

The water-soluble additive is preferably propylene glycol, sodium lauryl sulphate, one or more polaxomers, polyoxyl 35 castor oil, polyoxyl 40 hydrogenated castor oil, cetomacrogol, polyethylene glycol or transcutol; or any two or more of the above in combination.

When the composition is dispensed as an aerosol, the vehicle partly comprises a propellant in an amount to provide from 10% to 90% (w/w) of the composition.

The propellant can be any pharmaceutically acceptable propellant which provides a pressure of from 20 p.s.i. guage (137.9 kPa) to 130 p.s.i. guage (896.3 kPa) within an aerosol dispenser. Preferred propellants include hydrocarbons, for example, propane, butane, isobutane, or dimethylether; hydrofluorocarbons and hydrochlorofluorocarbons, for example dichlorodifluoromethane (P12), trichloromonofluoromethane (P11), dichlorofluoroethane, monochlorodifluoromethane (P22), dichlorotetrafluoroethane (P114), difluoroethane (P152 A), tetrafluoroethane (P134), heptafluoropropane (P227B); or compressed gases, for example, nitrogen or carbon dioxide.

The topical compositions of the present invention are quick drying, non-occlusive formulations which cause marked enhancement of the skin permeation of the drug both in vitro and in vivo when compared with transdermal patches. They offer the advantages of lower skin irritation, greater ease of use, increased dosage flexibility and a simpler method of manufacture when compared to existing transdermal patches.

The present compositions are a significant advance over conventional medicinal aerosol compositions, since they permit the application of a medicament by a method whereby no physical contact on the area of application is required, except by the film-forming spray itself. The topical films formed by the present compositions show excellent stability and peelability and can be easily removed from the site of application by washing with water.

The compositions are generally prepared by mixing the ingredients without liquefied propellant at a temperature of from 0°C to 100°C and at ambient pressure and then charging the resulting mixture together with the liquefied propellant into an aerosol dispenser to achieve the final composition. Mixing is preferably carried out at a temperature of from 10°C to 25°C. Alternatively, the mixed composition is placed in a pump dispenser, for example, a metered dose pump, which dispenses the composition typically without liquefied propellant since a pressurised atmosphere is not required. Propellant which is liquid at room temperature may, however, be included in a pump dispenser composition as part of the non-aqueous vehicle. The composition so prepared is sprayed from the dispenser on to a topical site, at which site it forms a stable, plastic film or patch.

The aerosol dispenser is preferably a conventional aerosol can having a conventional metered spray aerosol valve. The pump dispenser is preferably a conventional can or bottle having a conventional metered spray pump. Preferably, the aerosol dispenser has an all position valve having a shroud that permits spraying when the dispenser is held at any angle. In this way, horizontal bottom surfaces as well as horizontal top surfaces and vertical surfaces can be sprayed. The valve actuator can be any actuator which produces a spray and not a foam at the nozzle. A preferred valve actuator is a mechanical breakup actuator, which employs mechanical forces rather than expansion and evaporation of the propellant to produce a spray. A typical mechanical breakup actuator has a conical or cylindrical swirl chamber with an inlet channel oriented perpendicular to the axis thereof. This structure imparts a swirling motion to the aerosol mixture upon discharge. The swirling motion occurs around the axis of the swirl chamber forming a thin conical film of discharged mixture, which breaks into droplets as it leaves the swirl chamber and travels in the direction of the axis thereof. The result is a fine, soft, dispersed spray which can be easily controlled to produce a stable thin film of even thickness completely contacting the application site. In dispensing a composition of the invention the dispenser is typically held about I to 2 inches (2.5 to 5cm) from the application site and produces a film of even thickness. The dispensers used in the present invention are preferably compact units. They can be conveniently used for quick and easy application of a medicament over a large surface area. Typically, the area is not more than 50cm²; and is more preferably from 10cm² to 25cm².

The following Examples illustrate the preparation of compositions according to the present invention, in order that the invention may be more fully understood.

In general, a composition according to the present invention suitable for use in an aerosol dispenser can be prepared as follows:
1. Dissolve the film former in the chosen vehicle under stirring to form a clear solution.
2. Dissolve or suspend the active ingredient and solubilizer(s) along with the permeation enhancer, together with any water-soluble additives required, in the solution formed in step 1.
3. Add the plasticiser to the solution and fill a conventional aerosol can with the mixture.
4. Charge the filled can with liquefied propellant.

Examples of partly generalised formulas which can be used with any suitable medicament to prepare compositions according to the present invention for use in an aerosol dispenser include the following. In all of the generalised and specific Examples which follow below, it will be understood those Examples not indicated as containing dimethyl isosorbide are not strictly in accordance with the invention, although they are illustrative of basic compositions.

### Example 1

| Ingredients | Percent w/w |
|---|---|
| Active ingredient | 0.5 - 10.0 |
| Plastoid B | 2.25 |
| Eudragit E 100 | 0.25 |
| Propylene glycol | 3.0 |
| Sodium lauryl sulfate | 3.5 |
| Acetone | 20 |
| Propellant | q.s. |
| Vitamin E | 0.1 |
| Transcutol | 1.0 |

### Example 2

| Ingredients | Percent w/w |
|---|---|
| Active ingredient | 30 |
| PVP K-30 | 3 |
| Povidone VA-64 | 2 |
| Vitamin E | 0.5 |
| PEG 400 | 1.0 |
| Propylene glycol | 1.5 |
| Ethanol | 15 |
| Propellant | q.s. |

More specific examples of compositions for use in an aerosol dispenser include:

### Example 3

| Ingredients | Percent w/w |
|---|---|
| Active ingredient | 15 |
| Povidone | 3 |
| Povidone VA-64 | 2 |
| Vitamin E | 0.5 |
| Polyethylene glycol 400 | 1.0 |
| Propylene glycol | 1.5 |
| Ethanol | 15 |
| Acetone | 15 |
| Propellant | q.s. |

### Example 4

| Ingredients | Percent w/w |
|---|---|
| Estradiol | 1 |
| PVP K-30 | 6 |
| PVP VA | 4 |
| Vitamin E | 1 |
| Polyethylene glycol 6000 | 2 |
| Polyethylene glycol | 3 |
| Dichlorodifluoromethane (P12) | 58.1 |
| Trichloromonofluoromethane (P11) | 24.9 |

### Example 5

| Ingredients | Percent w/w |
|---|---|
| Estradiol | 2 |
| PVP K-30 | 6 |
| PVP VA | 4 |
| Vitamin E | 1 |
| Polyethylene glycol 6000 | 2 |
| Polyethylene glycol | 3 |
| Dichlorodifluoromethane (P12) | 24.9 |
| Trichloromonofluoromethane (P11) | 57.1 |

### Example 6

| Ingredients | Percent w/w |
|---|---|
| Alendronate sodium | 1 |
| PVPK-30 | 6 |
| PVP VA | 4 |
| Vitamin E | 0.5 |
| Menthol | 0.05 |
| Dimethyl isosorbide | 3.0 |
| Acetone | 10 |
| Ethanol | 10 |
| Tetrafluoroethane (P134) | 25.45 |
| Dicholorodifluoromethane (P12) | 40 |

To prepare a composition according to the present invention suitable for use in a pump dispenser the same general method of Example 1 can be used except that it is not necessary to charge the pump dispenser with liquefied propellant to provide a pressurised atmosphere. The mixture itself may contain propellant which is liquid at room temperature as part of the vehicle.

Examples of partly generalised formulas, which can be used with any suitable medicament to prepare compositions according to the present invention for use in a pump dispenser include:

### Example 7

| Ingredients | Percent w/w |
|---|---|
| Active ingredient | up to 30% |
| Plastoid B | 5.6 |
| Eudragit E 100 | 0.6 |
| Propylene glycol | 4.0 |
| Sodium lauryl sulfate | 3.0 |
| Acetone | 20 |
| Isopropyl alcohol | q.s. |
| Vitamin E | 0.2 |
| Transcutol | 2.0 |

### Example 8

| Ingredients | Percent w/w |
|---|---|
| Active ingredient | 0.5 - 10 |
| PVP VA | 10 |
| Vitamin E | 0.5% |
| Propylene glycol | 3 |
| Acetone | 15 |
| Ethanol | 25 |
| Trichloromonofluoromethane (P11) | q.s. |

More specific examples of compositions for use in a pump dispenser include:

### Example 9

| Ingredients | Percent w/w |
|---|---|
| Active ingredient | 25 |
| Povidone | 6 |
| Povidone VA-64 | 4 |
| Vitamin-E | 1.0 |
| Polyethylene glycol | 3 |
| Ethanol | 27 |
| Acetone | q.s. |
| Methylene chloride | 27 |

### Example 10

| Ingredients | Percent w/w |
|---|---|
| Active ingredient | 15 |
| PVP K 30 | 6 |
| PVP VA | 4 |
| Vitamin E TPGS | 0.5% |
| Dimethyl isosorbide | 5 |
| Ethanol | 20 |
| Trichloromonofluoromethane (P11) | q.s. |

### Example 11

| Ingredients | Percent w/w |
|---|---|
| Estradiol | 2 |
| PVP K-30 | 6 |
| PVP VA | 4 |
| Vitamin E | 1 |
| Polyethylene glycol 6000 | 2 |
| Polyethylene glycol | 3 |
| Acetone | 27 |
| Methylene Chloride | 27 |
| Ethanol | 28 |

### Example 12

| Ingredients | Percent w/w |
|---|---|
| Estradiol | 1 |
| PVP K-30 | 6 |
| PVP VA | 4 |
| Vitamin E | 1 |
| Polyethylene glycol 6000 | 2 |
| Polyethylene glycol | 3 |
| Acetone | 27 |
| Methylene Chloride | 28 |
| Ethanol | 28 |

With reference to the specific compounds of the above Examples, the following explanation is given. Eudragit E 100 is a self-adhesive, hydrophilic matrix system. It also acts as a solubilizer for the drug Estradiol.

Plastoid B is a film-former. When used together, Eudragit E 100 and Plastoid B give better peelability and water washability than when either is used alone.

Acetone is a volatile, quick-drying, non-occlusive vehicle which helps to dispense the contents of the spray over a large surface area.

Propylene glycol acts as a humectant to prevent the excessive drying of the application site after application of the medicament. It also acts as a plasticiser for the film formed after application. Propylene glycol additionally acts as a solubilizer for the drug and a permeation enhancer.

Propellant is necessary for developing proper pressure within the container and for expulsion of the composition when the valve is open. It is also responsible, together with the valve, for dispensing the product as a fine spray. The preferred propellants are very stable compounds and relatively nontoxic, inert and non-flammable.

Sodium lauryl sulfate acts as a solubilizer for the drug.

The compositions in the above Examples were discharged from the dispenser as a fine, soft dispersed spray which could be easily controlled to produce a stable thin film of even thickness on a target surface, for example a laboratory cover glass. The films have been observed to last for at least 24 hours. The concentrations of the film-formers in the composition can be varied as required to obtain a patch which can deliver the drug in a sustained manner for a period of up to 1 to 5 days. The film is easily removable from the application site by water in preparation for reapplication of the film or other treatment.

## Claims

1. A topical, medicinal spray composition comprising one or more medicaments in a volatile vehicle, one or more film-forming polymers in said vehicle, and from 1 to 10% (w/w) plasticiser, said plasticiser comprising dimethyl isosorbide, wherein the composition can be sprayed on a topical site to form a stable, breathable film on said site, from which film the medicaments are transdermally available.

2. A composition according to claim 1, which also contains a permeation enhancer.

3. A composition according to claim 2, which contains up to 30% by weight of said medicament(s), up to 8% by weight of said permeation enhancer and up to 90% by weight of said vehicle.

4. A composition according to claim 1, 2 or 3, which contains up to 15% by weight of said film-forming polymers.

5. A composition according to claim 1, comprising (w/w) from 0.1% to 25% of one or more medicaments, from 0.1% to 10% film-forming polymer and said vehicle q.s. 100%, and further comprising from 0.1% to 10% solubilizer and from 0.1% to 8% permeation enhancer.

6. A composition according to any of claims 1 to 5, wherein the amount of medicament is from 0.1% to 10%.

7. A composition according to any of claims 1 to 6, which further comprises from 1% to 7% (w/w) of one or more water-soluble additives.

8. A composition according to any of claims 1 to 7, wherein the medicament is anti-emetic, anti-anginal, anti-inflammatory, a steroid or a steroid hormone.

9. A composition according to any preceding claim, wherein said medicament is released from the said film either immediately or over a period of time.

10. A composition according to any preceding claim, wherein the medicament is scopolamine, nitroglycerine, clonidine, isosorbide dinitrate, propranolol hydrochloride, timolol maleate, clonazepam, verapamil, diclofenac sodium, naproxen sodium, ibuprofen, ketoprofen, indomethacin, piroxicam, ketorolac, tromethamine, nimesulide, hydrocortisone or esters thereof, dexamethasone, fluocinolone acetonide, betamethasone, estradiol, norethisterone, testosterone, progesterone, salbutamol, bambuterol, salmeterol xinafoate, fluticasone propionate, mometasone furoate, budesonide, beclomethasone dipropionate, sodium cromoglycate, isoprenaline sulphate; alendronic acid, pamidronic acid, etidronic acid, vasopressin, oxybutynin, imipramine, mitrazapine, desipramine, naratriptan, zolmitriptan, sumatriptan, nicotine, loperamide, misoprostol, hyoscyamine, atropine, trihexyphenidyl, lorazepam, diazepam, tiagabine, fluoxetine, paroxetine, lisinopril, trandolapril, captopril, amlodipine, felodipine, prazosin, amiloride, methamphetamine, sibutramine hydrochloride, nafarelin, leuprolide acetate, insulin, growth hormone and analogues thereof, doxazosin, tamsulosin, terazosin, finasteride, alprostadil, sildenafil, bromocriptine, cabergoline, selegiline, melatonin, glimepiride, rosiglitazone, glyburide or glipizide; any of the chiral forms of the above medicaments; pharmaceutically acceptable salts of any of the above; or any two or more of the above, including their chiral forms, in combination.

11. A composition according to any preceding-claim, wherein the film-forming polymer is any acrylic polymer or copolymer, preferably a non-ionic copolymer of methyl methacrylate and butyl methacrylate, a copolymer of dimethylamine ethyl methacrylate and a neutral methacrylic acid ester, ammonio methacrylate copolymer type B, ammonio methacrylate copolymer type A, methacrylic acid copolymer type A, methacrylic acid copolymer type B, or is polyvinyl acetate, cellulose acetate, polyvinyl alcohol, povidone, povidone vinyl acetate, copolyvidone, hydroxypropylmethyl cellulose, hydroxyethyl cellulose, methyl cellulose, or ethyl cellulose.

12. A composition according to claim 5 and any of claims 6 to 11, wherein the solubilizer is a copolymer of dimethylamine ethyl methacrylate and a neutral methacrylic acid ester; a surfactant, preferably a Tween, Span or sodium lauryl sulphate; a polyhydric alcohol, preferably propylene glycol or a polyethylene glycol; vitamin E, vitamin E TPGS (tocopheryl polethylene 1000 succinate), or labrasol; propylene carbonate; or any two or more of the above in combination.

13. A composition according to claim 5 and any of claims 6 to 12, wherein the film-forming polymer is a non-ionic copolymer of methyl methacrylate and butyl methacrylate and the solubilizer is a copolymer of dimethylamine ethyl methacrylate and a neutral methacrylic acid ester.

14. A composition according to any of claims 1 to 13, wherein in addition to the dimethyl isosorbide, the plasticiser also comprises one or more of triethyl citrate, acetyltributyl citrate, castor oil, propylene glycol or polyethylene glycol.

15. A composition according to claim 5 and any of claims 6 to 14, wherein the permeation enhancer is a lipophilic solvent, lyophilic solvent, preferably dimethyl sulfoxide, dimethyl formamide or isopropyl myristate; a surfactant, preferably Tween 80, menthol, or sodium lauryl sulfate; a two component system, preferably oleic acid and octyl dimethyl paraamino benzoic acid (Padimate O); menthol; mixed esters of capric and caprylic acids; or a polyhydric alcohol, preferably propylene glycol or transcutol; or any mixture of two or more thereof.

16. A composition according to claim 15, wherein the lipophilic solvent is dimethyl sulfoxide, dimethyl formamide or isopropyl myristate; and/or the polyhydric alcohol is propylene glycol, polyethylene glycol or transcutol.

17. A composition according to claim 7 and any of claims 8 to 16, wherein the water-soluble additive is propylene glycol, sodium lauryl sulphate, one or more polaxomers, polyoxyl 35 castor oil, polyoxyl 40 hydrogenated castor oil, cetomacrogol, polyethylene glycol or diethylene glycol monoethyl ether EP (transcutol); or any two or more of the above in combination.

18. A composition according to any preceding claim, wherein the vehicle comprises water or a non-aqueous solvent, wherein said solvent is preferably acetone, isopropyl alcohol, methylene chloride, methyl-ethylketone, absolute alcohol, ethyl acetate, trichloromonofluoromethane (P11), or methylene dimethyl ether.

19. A composition according to any preceding claim, wherein the vehicle comprises from 1% to 20% (w/w) of one or more humectants, preferably polyhydric alcohol(s) or polyvinyl pyrrolidone.

20. A composition according to claim 19, wherein the or each humectant is a polyhydric alcohol, preferably propylene glycol, butylene glycol, a polyethylene glycol, glycerol or sorbitol; or is polyvinylpyrrolidone.

21. A composition according to any preceding claim, wherein the vehicle partly comprises a propellant in an amount to provide from 10 to 90% of the composition.

22. A composition according to claim 21, wherein the propellant is a hydrocarbon, preferably propane, butane, isobutane, or dimethylether; a hydroflurocarbon or a hydrochloroflurocarbon, preferably dichlorodifluromethane (P12), trichloromonofluromethane (P11), dichlorofluroethane, monochlorodifluromethane (P22), dichlorotetrafluroethane (P114), difluroethane (P152 A), tetrafluoroethane (P134 A) or heptafluoropropane (P227 B); or a compressed gas, preferably nitrogen or carbon dioxide.

23. A dispenser containing a composition according to any of claims 1 to 22, which dispenser dispenses the composition as a spray.

24. A dispenser according to claim 23, wherein the composition is dispensed as a metered dose.

25. A pump dispenser according to claim 23 or 24, when dependent on any of claims 1 to 17.

26. An aerosol dispenser according to claim 23 or 24, when dependent on claim 21 or 22.

27. An aerosol dispenser according to claim 26, wherein the propellant provides a pressure of from 20 p.s.i. guage (137.9 kPa) to 130 p.s.i. guage (896.3 kPa) inside the dispenser.

28. An aerosol dispenser according to claim 26 or 27, wherein the valve of the dispenser is an all position valve.

29. An aerosol dispenser according to any of claims 26 to 28, wherein the dispenser has a mechanical break-up actuator.

30. A method of preparing an aerosol dispenser according to any of claims 26 to 29, which method comprises mixing the ingredients of the composition without propellant, and then charging the resulting mixture together with propellant into an aerosol dispenser.

31. A method of preparing a pump dispenser according to claim 25, which method comprises mixing the ingredients of the composition with or without liquid propellant, and then placing the mixed ingredients in a pump dispenser.

32. A composition according to any of claims 1 to 22 for use as a medicament.

## Patentansprüche

1. Topische medizinische Spray-Zusammensetzung, die ein oder mehrere Medikamente in einem flüchtigen Vehikel, ein oder mehrere filmbildende Polymere in dem Vehikel und 1 bis 10 (Gew.-) % Weichmacher umfasst, wobei der Weichmacher Dimethylisosorbid umfasst, wobei die Zusammensetzung auf eine topische Stelle unter Bildung eines beständigen atmungsaktiven Films auf der Stelle aufgesprüht werden kann, von welchem Film aus die Medikamente transdermal zur Verfügung stehen.

2. Zusammensetzung nach Anspruch 1, die außerdem einen Permeationsverbesserer enthält.

3. Zusammensetzung nach Anspruch 2, die bis zu 30 Gew.-% des bzw. der Medikamente(s), bis zu 8 Gew.-% des Permeationsverbesserers und bis zu 90 Gew.-% des Vehikels enthält.

4. Zusammensetzung nach Anspruch 1, 2 oder 3, die bis zu 15 Gew.-% des filmbildenden Polymers enthält.

5. Zusammensetzung nach Anspruch 1, die (in Gew.-%) 0,1 bis 25 % eines oder mehrerer Medikamente(s) , 0,1 bis 10 % filmbildendes Polymer und das Vehikel in einer Menge von q.s. 100% und des Weiteren 0,1 bis 10 % Lösungsvermittler und 0,1 bis 8 % Permeationsverbesserer umfasst.

6. Zusammensetzung nach einem der Ansprüche 1 bis 5, wobei die Menge des Medikaments 0,1 bis 10 % beträgt.

7. Zusammensetzung nach einem der Ansprüche 1 bis 6, die des Weiteren 1 bis 7 (Gew.-) % eines oder mehrerer wasserlöslicher Zusatzmittel umfasst.

8. Zusammensetzung nach einem der Ansprüche 1 bis 7, wobei das Medikament ein Antiemetikum, ein Mittel gegen Angina, ein Entzündungshemmer, ein Steroid oder ein Steroidhormon ist.

9. Zusammensetzung nach einem der vorhergehenden Ansprüche, wobei das Medikament aus dem Film entweder sofort oder über eine Zeitspanne freigesetzt wird.

10. Zusammensetzung nach einem der vorhergehenden Ansprüche, wobei das Medikament Scopolamin, Nitroglycerin. Clonidin, Isosorbiddinitrat, Propanololhydrochlorid, Timololmaleat, Clonazepam, Verapamil, Diclofenac-Natrium, Naproxen-Natrium, Ibuprofen, Ketoprofen, Indomethacin, Piroxicam, Ketorolac, Tromethamin, Nimesulid, Hydrocortison oder Ester desselben, Dexamethason, Fluocinolonacetonid, Betamethason, Estradiol, Norethisteron, Testosteron, Progesteron, Salbutamol, Bambuterol, Salmeterolxinafoat, Fluticasonpropionat, Mometasonfuroat, Budesonid, Beclomethasondipropionat, Natriumcromoglycat, Isoprenalinsulfat, Alendronsäure, Pamidronsäure, Etidronsäure, Vasopressin, Oxybutynin, Imipramin, Mitrazapin, Desipramin, Naratriptan, Zolmitriptan, Sumatriptan, Nicotin, Loperamid, Misoprostol, Hyoscyamin, Atropin, Trihexyphenidyl, Lorazepam, Diazepam, Tiagabin, Fluoxetin, Paroxetin, Lisinopril, Trandolapril, Captopril, Amlodipin, Felodipin, Prazosin, Amilorid, Methamphetamin, Sibutraminhydrochlorid, Nafarelin, Leuprolidacetat, Insulin, Wachstumshormon und Analoge desselben, Doxazosin, Tamsulosin, Terazosin, Finasterid, Alprostadil, Sildenafil, Bromocriptin, Cabergolin, Selegilin, Melatonin, Glimepirid, Rosiglitazon, Glyburid oder Glipizid, irgendeine der chiralen Formen der obigen Medikamente, pharmazeutisch akzeptable Salze irgend einer der obigen Substanzen oder irgendwelche zwei oder mehrere der obigen Substanzen, einschließlich der chiralen Formen, in Kombination ist.

11. Zusammensetzung nach einem der vorhergehenden Ansprüche, wobei das filmbildende Polymer ein Acrylpolymer oder -copolymer, bevorzugt ein nichtionisches Copolymer von Methylmethacrylat und Butylmethacrylat, ein Copolymer von Dimethylaminethylmethacrylat und einem neutralen Methacrylsäureester, Ammoniummethacrylat-Copolymer vom Typ B, Ammoniummethacrylat-Copolymer vom Typ A, Methacrylsäure-Copolymer vom Typ A, Methacrylsäure-Copolymer vom Typ B oder Polyvinylacetat, Celluloseacetat, Polyvinylalkohol, Povidon, Povidonvinylacetat, Copolyvidon, Hydroxypropylmethylcellulose, Hydroxyethylcellulose, Methylcellulose oder Ethylcellulose ist.

12. Zusammensetzung nach Anspruch 5 und einem der Ansprüche 6 bis 11, wobei der Lösungsvermittler ein Copolymer von Dimethylaminethylmethacrylat und einem neutralen Methacrylsäureester, ein Tensid, bevorzugt Tween, Span oder Natriumlaurylsulfat, ein mehrwertiger Alkohol, bevorzugt Propylenglykol oder ein Polyethylenglykol, Vitamin E, Vitamin E TPGS (Tocopherylpolyethylen 1000-Succinat) oder Labrasol, Propylencarbonat oder irgendwelche zwei oder mehrere der obigen Substanzen in Kombination ist.

13. Zusammensetzung nach Anspruch 5 und einem der Ansprüche 6 bis 12, wobei das filmbildende Polymer ein nichtionisches Copolymer von Methylmethacrylat und Butylmethacrylat und der Lösungsvermittler ein Copolymer von Dimethylaminethylmethacrylat und einem neutralen Methacrylester ist.

14. Zusammensetzung nach einem der Ansprüche 1 bis 13, wobei der Weichmacher zusätzlich zum Dimethylisosorbid auch eine oder mehrere unter Triethylcitrat, Acetyltributylcitrat, Rizinusöl, Propylenglykol oder Polyethylenglykol umfasst.

15. Zusammensetzung nach Anspruch 5 und einem der Ansprüche 6 bis 14, wobei der Permeationsverbesserer ein lipophiles Lösungsmittel, ein lyophiles Lösungsmittel, bevorzugt Dimethylsulfoxid, Dimethylformamid oder Isopropylmyristat, ein Tensid, bevorzugt Tween 80, Menthol oder Natriumlaurylsulfat, ein Zweikomponentensystem, bevorzugt Ölsäure und Octyldimethylparaaminobenzoesäure (Padimate O), Menthol, Mischester von Caprin- und Caprylsäure oder ein mehrwertiger Alkohol, bevorzugt Propylenglykol oder Transcutol oder eine Mischung von zwei oder mehreren derselben ist.

16. Zusammensetzung nach Anspruch 15, wobei das lipophile Lösungsmittel Dimethylsulfoxid, Dimethylformamid oder Isopropylmyristat und/oder der mehrwertige Alkohol Propylenglykol, Polyethylenglykol oder Transcutol ist.

17. Zusammensetzung nach Anspruch 7 und einem der Ansprüche 8 bis 16, wobei das wasserlösliche Zusatzmittel Propylenglykol, Natriumlaurylsulfat oder ein oder mehrere Polaxomere, Polyoxyl 35-Rizinusöl, gehärtetes Polyoxyl 40-Rizinusöl, Cetomacrogol, Polyethylenglykol- oder Diethylenglykolmonoethylether EP (Transcutol) oder irgend zwei oder mehrere der obigen in Kombination ist.

18. Zusammensetzung nach einem der vorhergehenden Ansprüche, wobei das Vehikel Wasser oder ein nichtwässriges Lösungsmittel umfasst, wobei das Lösungsmittel bevorzugt Aceton, Isopropylalkohol, Methylenchlorid, Methylethylketon, absoluter Alkohol, Ethylacetat, Trichlormonofluormethan (P11) oder Methylendimethylether ist.

19. Zusammensetzung nach einem der vorhergehenden Ansprüche, wobei das Vehikel 1 bis 20 (Gew.-) % eines oder mehrerer Feuchthaltemittel, bevorzugt mehrwertige(n) Alkohol(e) oder Polyvinylpyrrolidon umfasst.

20. Zusammensetzung nach Anspruch 19, wobei das oder jedes Feuchthaltemittel ein mehrwertiger Alkohol, bevorzugt Propylenglykol, Butylenglykol, ein Polyethylenglykol, Glycerin oder Sorbit oder Polyvinylpyrrolidon ist.

21. Zusammensetzung nach einem der vorhergehenden Ansprüche, wobei das Vehikel teilweise ein Treibmittel in einer Menge umfasst, die 10 bis 90% der Zusammensetzung bereitstellt.

22. Zusammensetzung nach Anspruch 21, wobei das Treibmittel ein Kohlenwasserstoff, bevorzugt Propan, Butan, Isobutan oder Dimethylether, ein Fluorkohlenwasserstoff oder ein Chlorfluorkohlenwasserstoff, bevorzugt Dichlordifluormethan (P12), Trichlormonofluormethan (P11), Dichlorfluorethan, Monochlordifluormethan (P22), Dichlortetrafluorethan (P114), Difluorethan (P152 A), Tetrafluorethan (P134 A) oder Heptafluorpropan (P227 B) oder ein Druckgas, bevorzugt Stickstoff oder Kohlendioxid, ist.

23. Spendegerät, das eine Zusammensetzung nach einem der Ansprüche 1 bis 22 enthält, welches Spendegerät die Zusammensetzung als Spray spendet.

24. Spendegerät nach Anspruch 23, wobei die Zusammensetzung als dosierte Dosis gespendet wird.

25. Pumpenspendegerät nach Anspruch 23 oder 24, wenn es von irgendeinem der Ansprüche 1 bis 17 abhängt.

26. Aerosolspendegerät nach Anspruch 23 oder 24, wenn es von Anspruch 21 oder 22 abhängt.

27. Aerosolspendegerät nach Anspruch 26, wobei das Treibmittel einen Druck von 137,9 kPa (20 psi) bis 896,3 kPa (130 psi) in dem Spendegerät bietet.

28. Aerosolspendegerät nach Anspruch 26 oder 27, wobei das Ventil des Spendegeräts ein Universalstellungsventil ist

29. Aerosolspendegerät nach Anspruch 26 oder 28, wobei das Spendegerät einen mechanischen Auftrennbetätiger aufweist.

30. Verfahren für die Herstellung eines Aerosolspendegeräts nach einem der Ansprüche 26 bis 29, welches Verfahren das Mischen der Bestandteile der Zusammensetzung ohne Treibmittel und das darauffolgendes Einfüllen der so erhaltenen Mischung mit dem Treibmittel in ein Aerosolspendegerät umfasst.

31. Verfahren für die Herstellung eines Pumpenspendegeräts nach Anspruch 25, welches Verfahren das Mischen der Bestandteile der Zusammensetzung mit oder ohne flüssigem Treibmittel und das darauffolgende Eingeben der gemischten Bestandteile in ein Pumpenspendegerät umfasst

32. Zusammensetzung nach einem der Ansprüche 1 bis 22 für die Verwendung als Medikament

## Revendications

1. Composition médicale pour atomisation locale comprenant un ou plusieurs médicaments dans un véhicule volatile, un ou plusieurs polymères formant un film dans ledit véhicule, et entre 1 et 10 % (en poids) de plastifiant, ledit plastifiant comprenant de l'isosorbide diméthylique, dans laquelle la composition peut être atomisée sur un site local pour former un film stable et respirable sur ledit site, tandis que les médicaments sont disponibles de manière transdermique à partir dudit film.

2. Composition selon la revendication 1, contenant également un stimulateur de pénétration.

3. Composition selon la revendication 2, contenant jusqu'à 30% en poids dudit (desdits) médicament(s), jusqu'à 8 % en poids dudit stimulateur de pénétration et jusqu'à 90 % en poids dudit véhicule.

4. Composition selon la revendication 1, 2 ou 3, contenant jusqu'à 15 % en poids desdits polymères formant un film.

5. Composition selon la revendication 1, comprenant (en poids) entre 0,1 et 25 % d'un ou de plusieurs médicaments, entre 0,1 et 10 % de polymère formant un film et ledit véhicule pour combler les 100 %, et comprenant en outre entre 0,1 et 10 % d'un agent de solubilisation et entre 0,1 et 8 % de stimulateur de pénétration.

6. Composition selon l'une quelconque des revendications 1 à 5, dans laquelle la quantité de médicament est comprise entre 0,1 et 10 %.

7. Composition selon l'une quelconque des revendications 1 à 6, comprenant en outre entre 1 et 7 % (en poids) d'un ou de plusieurs additifs solubles dans l'eau.

8. Composition selon l'une quelconque des revendications 1 à 7, dans laquelle le médicament est anti-émétique, anti-angine, anti-inflammatoire, un stéroïde ou une hormone stéroïde.

9. Composition selon l'une quelconque des revendications précédentes, dans laquelle ledit médicament est administré à partir dudit film, soit immédiatement soit sur une période temps donnée.

10. Composition selon l'une quelconque des revendications précédentes, dans laquelle le médicament est de la scopolamine, de la nitroglycérine, de la clonidine, du dinitrate d'isosorbide, de l'hydrochlorure de propranodol, du maléate de timolol ; du clonazépam, du vérapamil, du sodium de diclofénac, du sodium de naproxène, de l'ibuprofène, du cétoprofène, de l'indométhacine, du piroxicam, du kétorolac, de la trométhamine, du nimesulide, de l'hydrocortisone ou ses esters, du déxaméthasone, de l'acétonure de fluocinolone, du bétaméthasone, de l'estradiol, du noréthistérone, du testostérone, du progestérone, du sulbutamol, du bambutérol, du xinafoate de salmétérol, du propionate de fluticasone, du furoate de mométasone, du budésonure, du dipropionate de béclométhasone, du cromoglycate de sodium, du sulfate d'isoprénaline ; de l'acide alendronique, de l'acide pamidronique, de l'acide étidronique, de la vasopressine, de l'oxybutynine, de l'imipramine, de la mitrazapine, de la désipramine, du naratriptan, du zolmitriptan, du sumatriptan, de la nicotine, de la lopéramide, du misoprostol, de l'hyoscyamine, de l'atropine, du trihexyphénidyle, du lorazépam, du diazépam, de la tiagabine, de la fluoxétine, de la paroxétine, du lisinopril, du trandolapril, du captopril, de l'amlopdipine, de la félodipine, de la prazosine, de l'amiloride, de la méthamphétamine, de l'hydrochlorure de sibutramine, de la nafaréline, de l'acétate de leuprolide, de l'insuline, de l'hormone de croissance et ses analogues, de la doxazosine, de la tamsulosine, de la térazosine, de la finastéride, de l'alprostadil, du sildenafil, de la bromocriptine, de la cabergoline, de la sélégiline, de la mélatonine, du glimépiride, du rosiglitazone, du glyburide ou du glipizide ; n'importe quelle forme chirale des médicaments ci-dessus ; des sels pharmaceutiquement acceptables de n'importe lequel des médicaments ci-dessus; ou bien des combinaisons de deux des médicaments ci-dessus, ou davantage, y compris dans leur forme chirale.

11. Composition selon l'une quelconque des revendications précédentes, dans laquelle le polymère formant un film est n'importe quel polymère ou copolymère acrylique, de préférence un copolymère non ionique de méthacrylate méthylique ou de méthacrylate butylique, un copolymère de méthacrylate éthylique de diméthylamine et un ester d'acide méthacrylique neutre, un copolymère d'ammonio méthacrylate de type B, un copolymère d'ammonio méthacrylate de type A, un copolymère d'acide méthacrylique de type A, un copolymère d'acide méthacrylique de type B, ou bien de l'acétate de polyvinyle, de l'acétate de cellulose, de l'alcool de polyvinyle, du povidone, de l'acétate de povidone vinyle, du copolyvidone, de la cellulose d'hydroxypropylméthylique, de la cellulose hydroxyéthylique, de la cellulose de méthyle ou de la cellulose d'éthyle.

12. Composition selon la revendication 5 ou l'une quelconque des revendications 6 à 11, dans laquelle l'agent de solubilisation est un copolymère de méthacrylate éthylique de diméthylamine et un ester d'acide méthacrylique neutre ; un tensioactif, de préférence un Tween, Span ou sulfate laurylique de sodium ; un alcool polyhydrique, de préférence un propylène glycol ou un polyéthylène glycol ; de la vitamine E, de la vitamine E TPGS (du succinate de tocophéryle polyéthylène 1000) ou du labrasol ; du carbonate de propylène ; ou bien une combinaison de deux éléments ci-dessus quelconques ou davantage.

13. Composition selon la revendication 5 ou l'une quelconque des revendications 6 à 11, dans laquelle le polymère formant un film est un copolymère non ionique de méthacrylate méthylique ou de méthacrylate butylique, et l'agent de solubilisation est un copolymère de méthacrylate éthylique de diméthylamine et un ester d'acide méthacrylique neutre.

14. Composition selon l'une quelconque des revendications 1 à 13, dans laquelle outre l'isosorbide diméthylique, le plastifiant comprend également un ou plusieurs des éléments suivants: citrate triéthylique, citrate acétyltributylique, huile de ricin, propylène glycol ou glycol polyéthylène.

15. Composition selon la revendication 5 ou l'une quelconque des revendications 6 à 14, dans laquelle le stimulateur de pénétration est un solvant lipophile, un solvant lyophile, de préférence un sulfoxyde de diméthyle, un formamide de diméthyle ou un myristate d'isopropyle ; un tensioactif, de préférence Tween 80, du menthol ou du sulfate laurylique de sodium ; un système à deux composants, de préférence de l'acide oléique et de l'acide benzoïque octyle diméthylique paramino (Padimate O); du menthol ; des esters mélangés d'acide caprique et d'acide caprylique ; ou un alcool polyhydrique, de préférence un propylène glycol ou un transcutol ; ou tout mélange de deux éléments ci-dessus ou davantage.

16. Composition selon la revendication 15, dans laquelle le solvant lipophile est du sulfoxyde de diméthyle, du formamide de diméthyle ou du myristate d'isopropyle, et/ou l'alcool polyhydrique est du glycol propylène, du polyéthylène glycol ou du transcutol.

17. Composition selon la revendication 7 ou l'une quelconque des revendications 8 à 16, dans laquelle l'additif soluble dans l'eau est du propylène glycol, du sulfate laurylique de sodium, un ou plusieurs polaxomères, de l'huile de ricin 35 polyoxyle, de l'huile de ricin hydrogéné 40 polyoxyle, du cétomacrogol, du polyéthylène glycol ou de l'éther monoéthylique de diéthylène glycol EP (transcutol) ; ou une combinaison de deux éléments ci-dessus ou davantage.

18. Composition selon l'une quelconque des revendications précédentes, dans laquelle le véhicule comprend de l'eau ou un solvant non aqueux, dans laquelle ledit solvant est de préférence de l'acétone, de l'alcool isopropylique, du chlorure de méthylène, du méthyl-éthylcétone, de l'alcool absolu, de l'acétate d'éthyle, du trichloromonofluorométhane (P11) ou de l'éther diméthylique de méthylène.

19. Composition selon l'une quelconque des revendications précédentes, dans laquelle le véhicule comprend entre 1 et 20 % (en poids) d'un ou plusieurs agents humidifiants, de préférence un ou plusieurs alcools polyhydriques ou du pyrrolidone de polyvinyle.

20. Composition selon la revendication 19, dans laquelle l'agent humidifiant ou chaque agent humidifiant est un alcool polyhydrique, de préférence du propylène glycol, du butylène glycol, du glycérol ou du sorbitol ; ou bien du pyrrolidone de polyvinyle.

21. Composition selon l'une quelconque des revendications précédentes, dans laquelle le véhicule comprend partiellement un agent propulseur dans une quantité de nature à fournir entre 10 et 90 % de la composition.

22. Composition selon la revendication 21, dans laquelle l'agent propulseur est un hydrocarbure, de préférence du propane, du butane, de l'isobutane ou du diméthyléther ; un hydrofluorocarbure ou un hydrochlorofluorocarbure, de préférence du dichlorodifluorométhane (P12), du trichloromonofluorométhane (P11), du dichlorofluoroéthane, du monochlorodifluorométhane (P22), du dichlorotétrafluoroéthane (P114), du difluoroéthane (P152 A), du tétrafluoroéthane (P134 A) ou de l'heptafluoropropane (P227 B) ; ou du gaz comprimé, de préférence de l'azote ou du dioxyde de carbone.

23. Dispensateur contenant une composition selon l'une quelconque des revendications 1 à 22, lequel dispensateur distribue la composition sous forme d'atomiseur.

24. Dispensateur selon la revendication 23, dans lequel la composition est administrée sous forme dosée.

25. Dispensateur à pompe selon la revendication 23 ou 24, dans le cadre de l'une quelconque des revendications 1 à 17.

26. Dispensateur aérosol selon la revendication 23 ou 24, dans le cadre de la revendication 21 ou 22.

27. Dispensateur aérosol selon la revendication 26, dans lequel l'agent propulseur fournit une pression compris entre 20 psi (137,9 kPa) et 130 psi (896,3 kPa) à l'intérieur du dispensateur.

28. Dispensateur aérosol selon la revendication 26 ou 27, dans lequel la valve du dispensateur est une valve universelle.

29. Dispensateur aérosol selon l'une quelconque des revendications 26 à 28, dans lequel le dispensateur possède un actionneur à rupture mécanique.

30. Procédé de préparation d'un dispensateur aérosol selon l'une quelconque des revendications 26 à 29, lequel procédé consiste à mélanger les ingrédients de la composition sans agent propulseur, puis à charger le mélange résultant avec l'agent propulseur dans un dispensateur aérosol.

31. Procédé de préparation d'une pompe à dispensateur selon la revendication 25, lequel procédé consiste à mélanger les ingrédients de la composition avec ou sans agent propulseur liquide, puis à verser les ingrédients mélangés dans un dispensateur à pompe.

32. Composition selon l'une quelconque des revendications 1 à 22 pour utilisation comme médicament.
